# EUROPEAN PATENT SPECIFICATION

(11) **EP 0 915 668 B1**
(45) Date of publication and mention of the grant of the patent: **13.03.2002**
(21) Application number: 97934795.2
(22) Date of filing: 06.08.1997
(51) Int. Cl.: A23L 1/305, A23C 9/13, A23L 2/66, A61K 38/38

(54) **DRINKABLE, ACID NUTRIENT COMPOSITION WITH LONG SHELF LIFE AND HEALTH-PROMOTING ACTION**
LAGERFÄHIGE TRINKBARE SAUERE NÄHRUNGSZUSAMMENSETZUNG ZUR VERBESSERUNG DER GESUNDHEIT
COMPOSITION BUVABLE, NUTRITIVE, ACIDE, LONGUE CONSERVATION ET BONNE POUR LA SANTE

(30) Priority: 06.08.1996 NL 1003748
(43) Date of publication of application: 19.05.1999
(73) Proprietor: N.V. Nutricia, 2700 MA Zoetermeer (NL)
(72) Inventor: SNOEREN, Thomas, Hubertus, Martinus, NL-8334 NE Steenwijk (NL); GOETHALS, Maud, NL-3512 KG Utrecht (NL)
(74) Representative: de Bruijn, Leendert C.
(86) International application number: NL9700457
(87) International publication number: WO9805221

(56) References cited:
- EP-A- 0 029 370
- EP-A- 0 211 118
- DE-A- 2 202 267
- GB-A- 2 116 819
- US-A- 3 737 326
- US-A- 5 455 054
- PATENT ABSTRACTS OF JAPAN vol. 010, no. 094 (C-338), 11 April 1986 & JP 60 224467 A (SETOUCHI KOGYO KK), 8 November 1985, cited in the application
- DATABASE WPI Section Ch, Week 8551 Derwent Publications Ltd., London, GB; Class D13, AN 85-320705 XP002028269 & JP 60 224 467 A (SETOUCHI KOGYO KK) , 8 November 1985
- CHEMICAL ABSTRACTS, vol. 104, no. 15, 14 April 1986 Columbus, Ohio, US; abstract no. 128647z, TAKAHATA J.: "Vinegar-pickled eggs as health drinks" page 597; column 2; XP002028268 & JP 60 224 467 A

## Description

The present application relates to a liquid, acid nutrient composition with a long shelf life. More particularly, the application relates to a composition of this type which has a health-promoting action.

More particularly, the present application relates to a fermented, liquid nutrient composition with a long shelf life, in the form of a drinkable yoghurt-type preparation, on the basis of "egg-white protein" (EWP).

Nutrient preparations having a health-promoting action are used in health care as a supplement to the daily food or as a replacement for the latter.

However, the commercially available formulations in general have a poor taste or other poor characteristics with regard to consumption. This is a disadvantage in particular for seriously and/or chronically ill patients who are dependent on such nutrient preparations, and also for children.

A first aim of the invention is to provide a nutrient composition having a health-promoting action, which composition has a pleasant taste (or a variety of tastes) and can be consumed easily, even by patients having a very poor appetite or with disorders of the digestive tract, including impairment of the jaw (muscles), the throat and/or the swallowing reflex.

Patents Abstracts of Japan, vol.10, no. 094 (C-338); Derwent Abstract AN 85-320705 en Chemical Abstracts 1986, vol. 104, abstr. no. 128647z (all corresponding to Japanese patent application 60 224 467) describe a drinkable egg vinegar syrup, which is prepared by pickling an egg in vinegar or acetic acid until its shell is completely dissolved, and then adding citric acid, optionally in combination with fruit juice, to egg vinegar to the pickling solution. The mixture thus obtained is homogenized and its pH is adjusted to 2,1 - 4,3. In this way, a calcium rich health drink is obtained, in which the bitter taste of egg vinegar ( due to calcium and similar compounds from the dissolved eggshell) is masked by the citric acid and the fruit juice.

US-A-3,737,326 describes a beverage containing egg albumin and whey as a source of protein, and optionally sweetener, color, flavour and/or food acid. The food acid is present in an amount to attain a pH from about 2.5 to 4.6 in the final product, which enables the whey, which constitutes from about 20% to about 50% of the total of the wheylalbumine mixture, to inhibit or eliminate the taste and odor of the egg albumin in the beverage. The final product simulates natural fruit or vegetable beverages; preparation of a fermented product, in particular of a drinkable yoghurt-type product, is neither described nor suggested.

GB 2 116 819 A describes a cultured egg-milk product, prepared by combining 65-85 % by weight whole or skim cow's milk and up to 20% by weight liquid, whole or powdered egg and fermenting said mixture using a suitable bacterial culture such as the LB₁-strain of L. Bulgaricus and the TS₂-strain of S. Thermophilus. Preferably, a stabilizer is added to the pre-fermentation composition in an amount of 0.1-1 % by weight so as to provide the product with a firmer body. Furthermore, 2-10% by weight sugar can be added.

EP-A-0 211 118 describes drinking preparations having both whole egg and fruit juice (typically about 35%) as constituents. The use of EWP is mentioned nor suggested. It is mentioned that "Under conditions of low pH such as are typically found in fruit juices the protein in whole egg precipitates out into an unsightly precipitate or sediment. A similar effect occurs when albumin, the protein of egg white, is heated."

Therefore, in order to improve the shelf-life and homogeneous texture of the product, the whole egg/fruit juice mixture is treated so that the precipitate resulting from combining the juice and egg remains in the form of finely divided particles evenly dispersed throughout the liquid medium. i.e. by homogenisation. The preparation of a fermented product is neither described nor suggested.

US-A-2,919,195 describes an acidic fruit juice enriched with protein, in particular milk protein, which consists essentially of a dispersion in a fruit juice of a blend of a water-dispersable, naturally occuring protein selected from casein, milk protein and soy protein, with a pH of about 3,5-4,5. This composition of necessity contains 0,3 to 1 part egg yolk per part protein in order to prevent precipitation of the protein at the final pH. The product is prepared by adding the egg-yolk to the protein base and homogenizing at a temperature below the heat denaturation temperature of the egg yolk. The homogenized mixture is then added to the acidic fruit juice to give a dispersion, which is further homogenized, after which the pH is adjusted, if required, to a pH of about 3,5 to 4,5 using a food grade acid.

The abovementioned products are all prepared on the basis of whole egg, which is required because of its properties as an emulgator/stabilizer, i.e. in order to prevent precipitation of the protein which occurs at the low pH of the final product. However, the use of whole egg has certain disadvantages, such as:
- whole egg has a relatively high cholesterol content and a relatively low conalbumine content, compared to egg white protein;
- whole egg is more difficult to handle in a technological process for food preparation, compared to EWP. For instance, there is the constant risk of curdling of the lipids from the egg yolk;
- the use of whole egg can give the final product a strong egg-like taste or smell, which is mostly due to the components from the egg yolk. Also, the presence of egg yolk components can give the product an undesirable egg-like appearance (i.e. a yellowish colour).

EP-A-0 029 370 describes a protein product with improved acid and temperature stability prepared from whey, which can be used as a replacement for egg albumin in food products, such as acidic beverages, because of its bland flavour.

US-A-5,455,054 describes a method for preparing liquid, pasteurized egg compositions with improved stability based principally on egg whites.

DE-OS-22 02 267 describes a "soft drink-like" protein drink, which has been adjusted to a pH below the iso-electric point of the protein using a food grade acid.

It is known that certain constituents of milk have a beneficial effect on health. One example of such constituents is lactoferrin, which is known to have a beneficial action with regard to bacterial disorders, such as infections of the digestive tract, and also viral infections.

However, the lactoferrin in cows' milk has too low an activity to be really effective in the quantities in which it occurs in cows' milk.

Furthermore, the preparation and/or isolation of lactoferrin, and in particular of human lactoferrin, are laborious and expensive, with the result that the addition of (isolated) lactoferrin to milk is not advantageous from the economic standpoint.

A second aim of the invention is, therefore, to provide a nutrient composition which can be prepared simply from generally available, inexpensive raw materials and which has improved characteristics with regard to the promotion of health, compared with (cows') milk alone.

To this end, according to the invention, a nutrient composition is provided which in addition to milk contains the "egg-white protein" (EWP) from birds' eggs, in particular hens' eggs.

The use of EWP in food products is known. The known uses are, in particular, functional applications of the processed protein, for example to provide a firm or foam-like (stable) structure in cakes and pastries, puddings or similar products. The use of, in particular, un-denatured EWP in nutrient formulations having a health-promoting action has not been described.

Furthermore, the use of EWP in liquid products has a number of disadvantages:
- In the liquid (non-processed or "raw") form, EWP has poor characteristics in respect of odour and taste, as well as the processing thereof.
- Constituents of eggs, such as EWP, usually contain germs of undesired microorganisms.
- Liquid preparations based on EWP generally have a very short shelf life, that is to say a shelf life of less than one month, or mostly less than one week.

For these reasons virtually no long-shelf life liquid preparations based on EWP have been described in the prior art. The only commercially available liquid EWP preparation which is said to have a shelf life of more than one month (ROGRès from LIOT) is intended as a constituent for processing in other products and is unsuitable for direct consumption, inter alia because of the poor taste and a pH of over 9.0. Moreover, ROGRès from LIOT is not fermented.

The long shelf life of ROGRès® is linked to the high pH thereof; when ROGRès® is acidified to a pH in the range of 5 - 7 its keeping qualities are lost and pathogens present in the preparation are able to develop.

The keeping qualities of liquid EWP-containing preparations could, in theory, be improved by sterilisation, for example by heat treatment at above 135 - 140 °C, such as in a UHT sterilisation. However, this has the disadvantage that the EWP coagulates, as a result of which the liquid character of the EWP is lost and the appearance and the taste of the product deteriorate to an unacceptable extent. Furthermore, the active constituents of the EWP are largely denatured, as a result of which the health-promoting action can be lost. In this respect EWP is more sensitive to high temperatures than (the protein-like constituents of) egg yolk: "egg-white" starts to denature at about 60 °C, whereas "egg yolk" is essentially stable to heat up to temperatures of 80 °C or above.

A third aim of the invention is therefore to provide a nutrient composition based on EWP which has a long shelf life, without the disadvantages which are associated with a sterilisation treatment.

To this end the invention provides, in a first aspect, a liquid, acid nutrient composition with long shelf life, which contains 0.1 - 5 % by weight "egg-white protein" (EWP), based on the total composition, wherein less than 40 %, preferably less than 10 % and most preferentially less than 2 % of the EWP is denatured.

In a further aspect, the invention relates to the use of EWP or a product derived from EWP, preferably in the dried form or in the form of a spray-dried preparation, in the preparation of a liquid, acid nutrient composition with long shelf life and health-promoting action.

"Egg-white protein" (EWP) is understood to mean all the proteins and further constituents from the egg-white of birds' eggs (as opposed to the yolk), in particular of hens' eggs, although the invention is not restricted to the latter.

In the invention said EWP is preferably used in the form of a dried or spray-dried preparation which contains the proteins in a (virtually) non-denatured form. Said preparations are preferably sterile.

However, it is also possible to use a non-sterile preparation or the abovementioned ROGRès® ; however, the latter must not be kept at a pH in the range of 5 - 7 for a sufficient time for any germs present to be able to develop. Finally, EWP obtained direct from eggs can also be used, as such or after a further treatment, such as pasteurisation.

The abovementioned dry EWP preparations can be obtained in a manner known per se by (spray) drying the "raw" egg-white after separating off the yolk and any further undesired constituents. In addition to the further advantages mentioned in this description, the use of a dried/spray-dried EWP has the advantage that it can be processed appreciably better than the liquid "egg-white protein" itself or similar products, such as liquid egg; thus, the occurrence of curdling during preparation will be counteracted.

Furthermore, suitable products derived from EWP, such as fractions and/or concentrates of EWP, or, for example, a conalbumin concentrate, can also be used. However, compared to such specific isolates, the use of EWP has the advantage that it contains further advantageous constituents, such as glycoproteins and lysozyme. Also, EWP is in general easier to prepare than these isolates.

A very suitable spray-dried preparation is available commercially from NIVE under the name "Kipeiwitpoeder".

The EWP used as starting material must in any event be such that ultimately a nutrient composition is obtained in which less than 40 %, preferably less than 10 % and most preferentially less than 2 % of the EWP is denatured.

The EWP is used in amounts of 0.1 - 5 % by weight, preferably 0.5 - 3 % by weight, based on the total composition, and preferably in an amount such that a conalbumin concentration of 0.01 - 0.7 % by weight is obtained in the liquid end product, depending on the starting material used. If necessary, a/an (additional amount of) conalbumin concentrate can also be added for this purpose. Preferably, the EWP concentration will be below 2 % by weight in order to guarantee the pleasant taste of the product; in the case of preparations containing more than 5 % EWP the adverse effects on the taste can start to play a major (too great a) role.

According to the invention the EWP is preferably mixed with milk or a product derived from milk, after which the mixture thus obtained is rendered acid/acidified and pasteurised. A liquid, acid milk-based nutrient composition is thus obtained which has a long shelf life and in which the EWP is (virtually) not denatured.

With this method the milk from any suitable mammal, such as cows' milk, can be used. It is also possible to use mixtures of milk from different species of mammal, such as mixtures of cows' milk and goats' milk, for example mixtures containing more than 50 %, preferably more than 80 % goats' milk. The milk used can be full-fat, semi-skimmed or skimmed milk, to which additional milk fat and other components such as vitamins, minerals and trace elements can optionally also be added.

Furthermore, suitable products derived from milk can also be used, such as skimmed milk powder (optionally with added milk fat) or whey powder, as well as milk fractions obtained by, for example, ultrafiltration, provided said fractions can be acidified/fermented in a manner known per se.

For the preparation of nutrient compositions according to the invention having a health-promoting action preference is given to full-fat goats' milk, as will be explained in more detail below.

However, it is also possible to mix the EWP with a prefermented/ acidified milk product, or with another acid base which is suitable for the preparation of a drinkable formulation, in particular acid fruit juices, such as orange juice, grapefruit juice and the like, an EWP-containing fruit drink being obtained. Mixtures of a (fermented or unfermented) milk base and fruit juice can also be used.

The composition of the invention can also contain all suitable constituents acceptable for foods, depending on the desired product and the ultimate intended use.

For instance, it is possible to incorporate flavourings and aroma substances, such as natural fruit aromas. In this way it is possible to obtain a pleasant taste or a variety of tastes, comparable to commercially available dairy products.

Furthermore, it is also possible to incorporate sugars, vitamins (such as biotin, riboflavin, B12), fibres (inulin), amino acids, oils and fats/fatty acids, trace elements (such as Cr, Mn, Zn) and sources of Ca, P, Na, K, Mg and the like in suitable amounts.

When the compositions of the invention are in the form of food supplements, they will in any event contain sugars, vitamins and trace elements, although this is not a limitation, in amounts which are dependent on the ultimate intended use, as well as on the opinion of the expert.

Complete foods will contain a broader spectrum of constituents, in general all constituents which are required to meet human food needs, in amounts which correspond to the recommended daily allowance (RDA), as will be known to those skilled in the art.

It is optionally possible also to incorporate colourings and the like, as well as stabilisers. For the preparation of a yoghurt drink, in particular pectin will be added in a customary amount of about 0.1 - 2 % by weight.

When a food supplement type formulation is prepared, such as a fermented drinkable yoghurt, the various constituent starting materials will therefore be incorporated in the following amounts:
- protein (total, including EWP constituent): 1 - 10 % by weight;
- milk constituent: 99.9 - 60 % by weight, preferably 80 - 95 %;
- other constituents: 0 - 20 % by weight, preferably 2 - 17 %;
the total of all constituents making up 100 % by weight.

When the composition of the invention is in the form of a formulation based on a based on a fruit or vegetable extract, it will generally contain:
- protein (total, including EWP constituent): 0.1 - 8 % by weight;
- carbohydrates: 1 - 20 % by weight, preferably 2 - 8 %
with water and the other constituents mentioned herein, making up 100 %

When the composition of the invention is in the form of a complete food formulation, it will generally contain:
- protein (total, including EWP constituent): 2 - 10 % by weight;
- fats and oils: 2 - 6 % by weight;
- carbohydrates: 8 - 16 % by weight;
with water and the other constituents mentioned herein, such as minerals, trace elements and other desired additives (which can be present in amounts which correspond to the recommended daily allowance (RDA)) making up 100 %. The complete food formulations can for instance also contain fibres, in amounts of 0 - 4 % by weight.

In all the abovementioned compositions, the amount of EWP component will be in the range of 0.1 - 5 % by weight, preferably 0.5 - 3 % by weight, of the total composition.

The compositions of the invention preferably contain essentially no egg yolk components (such as egg yolk proteins etc.), i.e. less than 5 % by weight, more preferably less than 0.5 % by weight, based on total protein, which is achieved by using starting materials derived from egg white. Nevertheless, if desired, egg yolk components can probably be incorporated in amounts of up to 15 % by weight of total protein, without adversely affecting the properties, and in particular the taste, of the final product. These amounts are still substantially lower than those that would be present if whole egg were to be used in the preparations of the compositions of the invention, as whole egg generally contains 40 - 45 % by weight egg yolk proteins, based on total protein. Also, according to the invention, the presence of egg yolk for its stabilizing properties as described in the abovementioned art is not required.

When the composition of the invention is in the form of a fermented product, it can contain whey, derived from the milk constituent used as the starting material in the preparation thereof. In these cases, the whey proteins will generally be present in usual amounts for fermented milk-type products, i.e. 15 - 20 % by weight of total protein.

When the composition of the invention is in the form of a complete food, it can also contain whey proteins, in amounts of 0 - 30 % by weight, preferably 15 - 20 % by weight, of total protein.

It is, however, also possible to use whey-derived preparations as a starting material and/or an additional protein source (besides the EWP), when preparing the fermented compositions or total food compositions of the invention. In these cases, the amount of whey or whey proteins will generally be higher, and can be more than 50 % by weight of total protein.

The fruit or vegetable extract-based compositions of the invention will generally contain essentially no whey proteins, i.e. no more than 10 % by weight, preferably less than 5 % by weight, more preferably less than 1 % by weight, based on total protein. However, if desired, whey can be incorporated in larger amounts, for instance in amounts up to 20 % by weight. These amounts are still far less then those required in the fruit-type drink of US patent 3,737,326. Also, according to the invention, the presence of whey is not required to mask the egg-like taste of the final product.

The composition of the invention is very highly preferentially drinkable, that is to say it has a viscosity and consistency which render it suitable for use as a drink and/or as a formulation for administration by tube or catheter, especially into the stomach. For this purpose the product will in general have a viscosity of less than 500 cPs, preferably less than 100 cPs, and more preferentially less than 50 cPs (at 20 °C), as measured in a standard instrument at a shear rate of 100 s⁻¹. This is in contrast to a yoghurt, which at a pH of <4.5 has a viscosity of more than 500 cPs.

The composition of the invention is an acid preparation, that is to say it has a pH of less than 5.0, more particularly less than 4.5, and usually 4,0 - 4,2, which is essential to obtain the required keeping quality.

To this end, the mixture of the starting materials can, if required, be (further) acidified, for example by adding acids acceptable for foods, such as citric acid, or constituents which contain said acids, such as fruit juices or fruit juice concentrates. The final pH must be acceptable for food products and must not be so low that the constituents of the product are affected or denatured.

Acidification of milk-based compositions according to the invention is preferably carried out by fermentation, in particular by means of the generally known action of lactic acid bacteria on the milk constituent with the formation of lactic acid, for which purpose suitable lactic acid bacteria such as L. bulgaricus, L. lactis or S. thermophilus are used, under conventional conditions for the preparation of fermented (dairy) products, such as, for example, are described in Bulletin of the International Dairy Federation (IDF), number 202 (1986) and 277 (1992).

With this procedure the nutrient composition according to the invention is obtained in the form of a drinkable fermented acid dairy product, such as a yoghurt drink, depending on the further processing. Said fermented product will have a pH in the range customary for fermented dairy products of this type, that is to say 3.5 - 5.0, more particularly 4.0 - 4.3, depending on the degree of acidification. The fermented product can, if necessary, be further acidified using the abovementioned acids/acid constituents.

It is also possible first to prepare a milk-based fermented acid composition, to which the EWP is then added in the abovementioned amounts, if necessary followed by further acidification and/or homogenisation.

The acid nutrient composition of the invention can also be non-fermented, for example in the form of a complete food, such as a food for administration by tube or catheter into the stomach, or in the form of an EWP-containing fruit drink, as described above.

After the suitable pH has been obtained, further desired constituents can be incorporated. The mixture is then homogenised and pasteurised under conventional conditions, for example by exposing it to a temperature of 70 °C for 30 minutes, or of 90°C for 30 sec. The EWP undergoes virtually no denaturing as a result of this pasteurisation. Furthermore, it must be understood that a possible sterilisation procedure without this resulting in denaturing of the EWP also falls within the scope of the invention.

The composition of the invention which is thus obtained can be considered as belonging to the category of long-shelf life products, that is to say it has a shelf life of at least one month, preferably at least 6 months, when it is stored at a temperature of between about 4 °C and about room temperature. Such keeping qualities, which are of very great importance for commercial food products, and in particular for health-promoting nutrient compositions, have not previously been achieved for EWP-based liquid nutrient compositions which are suitable for direct human consumption.

The invention will be explained in more detail below with reference to nutrient compositions having a health-promoting action. However, it must be understood that the invention can be employed in general for the preparation of EWP-based drinkable food products which have a long shelf life and a pleasant taste and that the invention is not particularly restricted to a specific end use of the products obtained.

It must also be understood that the composition of the invention can be in the form of a dry preparation, in particular in the form of a powder, which is suitable for providing a liquid composition according to the invention after reconstitution. A dry preparation or concentrate of this type can be prepared, using the liquid composition of the invention as starting material, by means of a suitable process known per se, such as drying or spray drying, for example as is used in the preparation of milk powder or concentrates.

The compositions of the invention have a health-promoting action and to this end can be used as a food supplement, as a complete food or as a food for administration by tube or catheter, i.e. into the stomach.

In this context the compositions of the invention, in particular in the form of a fermented dairy product, have the advantage of a liquid, and in particular drinkable, form, which facilitates consumption by the patient or the administration thereof to the patient. Furthermore, the compositions of the invention have a pleasant, natural taste comparable to that of conventional fruit drink or dairy products.

The compositions of the invention also have the advantage of a broad therapeutic and/or preventive efficacy as described in more detail below, without it being necessary for this purpose to add to the food specific, previously isolated active substances, which, moreover, can have adverse side effects. This means that the compositions of the invention can be generally used without the overall formulation thereof having to be specifically adjusted to the patient/user, although the incorporation of further therapeutically active constituents in the compositions is not precluded.

The nutrient according to the invention works well in the prevention and treatment of viral infections and bacterial infections, for example when the host is not sufficiently able to render these harmless him or herself; the composition also improves the immune function and has a regulatory action in disease processes mediated by free radicals.

The compositions of the invention are therefore also very suitable for use by people who have a weakened or inadequately developed defence system, such as babies, the elderly and the ill, or, for example, after operations or in patients undergoing chemotherapy. The invention is particularly suitable for use with AIDS patients and patients with CMV infections.

Furthermore, the invention can advantageously be employed in the case of patients suffering from disorders of the digestive tract, such as stomach/intestinal infections, Crohn's disease and the like, as well as other disorders, which may or may not involve the digestive tract, which render patients dependent on adapted and/or complete foods as a replacement for or a supplement to ordinary food.

The compositions of the invention can also have a beneficial action on oral infections/aphthae and on osteoporosis.

In the prior art it has been attempted to combat/prevent the abovementioned disorders and to improve the immune function by means of foods and/or supplements. However, none of the known products offers an adequate solution in the form of a long-shelf life, drinkable product which patients find pleasant to the taste.

The compositions of the invention can also be used as fortifying food, both in the case of illness and in the case of a high level of exertion, such as in sport, the compositions combining a good, natural taste with a high content of useful proteins, oligosaccharides and cysteine/glutathione precursors.

In the compositions of the invention, in particular the fermented preparations based on dairy products and more particularly the compositions based on EWP and goats' milk, the health-promoting properties of the chosen ingredients complement one another in respect of essential aspects, it being possible to obtain a synergistic effect. In many cases the combination of constituents will be essential in order to obtain a beneficial effect on the disorder to be treated/prevented. For instance, in the case of an AIDS patient who is suffering from a bacterial infection the cause of said infection can be a poorly functioning immune system, inadequate intestinal function or a combination thereof. By the use of the combination of ingredients according to the invention, an improvement is obtained in both groups of patients and/or both types can be treated in one patient, without intensive clinical investigation having to be carried out to determine the cause of the illness (insofar as this can be determined).

However, the invention is not restricted in any way whatsoever, neither in respect of the possible effectiveness nor in respect of the mechanism responsible for this.

Furthermore, the local action of biologically active proteins in the intestine, but also the biological availability of peptides (and thus of the individual amino acids) are dependent on the configuration of the molecule. This configuration is partly determined by the heat treatment to which the molecule is subjected: heat treatment at too high a temperature will cause the molecule to "denature", as a result of which the active substances will be less effective.

With the conventional method for the preparation of liquid milk- or egg-based nutrient compositions, intensive heating steps are often used in order to obtain a microbiologically safe product with a long shelf life.

According to the invention, heating of this type is avoided by making use of a low pH in the product in combination with gentle pasteurisation. In the compositions of the invention these active constituents, and in particular the proteins, are therefore present in a non-denatured form.

To summarise, according to the invention a composition is obtained which has a long shelf life and a pleasant taste, as well as a broad spectrum of therapeutic efficacy, and which can be obtained using inexpensive, natural constituents as starting materials.

The invention will now be explained with the aid of the following examples.

### Example I

### Preparation of a liquid supplement in the form of a yoghurt drink

1 % spray-dried EWP is added to full-fat goats' milk. After inoculation with desired microorganisms (lactic acid bacteria), the mixture is fermented at 37 °C until the pH is 4.8. After pasteurisation and homogenisation, 6 % sugar, 2 % fruit concentrate and 0.3 % pectin are then added, after which the mixture has a pH of 4.2. The pasteurised product is filled into containers under aseptic conditions.

In this composition it is also possible to use, instead of full-fat goats' milk, cows' milk or a mixture of goats' and cows' milk, or skimmed goats' milk to which milk fat is added; a composition of this type can optionally be homogenised prior to fermentation.

### Example II

### Liquid supplement based on fruit juice

2.5 % sugar, 0.35 % pectin and also sources of biotin, riboflavin, Ca, Cu, Mn and Zn are added to a solution of 1 % EWP. This bulk liquid is pasteurised for 20 sec at 70 °C and then homogenised in two steps (under 350 and 50 bar). 20 % fruit concentrate is added to the product thus obtained, after which the complete mixture is filled under aseptic conditions into containers which are subsequently pasteurised.

### Example III

### Preparation of a liquid complete food, for administration by tube, based on hydrolysed whey protein

1 % EWP is added to a solution of hydrolysed whey protein, after which the mixture is fermented with suitable lactic acid bacteria at 37 °C until a pH of about 4.1 has been obtained. The other ingredients are then added, including vegetable oils, maltodextrin, vitamins, minerals, trace elements, suitable sources of Ca, P, Na, K, Mg, as well as pectin and an emulsifier, after which the mixture is homogenised and pasteurised. The final mixture is filled into the final packaging under aseptic conditions.

### Example IV

### Preparation of a liquid product based on caseinate.

Per 100 l water are dissolved:
2.6 kg calcium caseinate,
2.6 kg sodium caseinate
1.9 kg eggwhite protein
4.3 kg saccharose.
Furthermore 6.3 kg canola high oleic acid and 0.35 kg lecithin are added. The mixture is pasteurized (10-25 sec., 63-70°C) and homogenized (55-65°C, 250-350/30-60 bar), and cooled to 35-40°C after which 0.9 kg of a yoghurt culture is added. The mixture is then fermented under stirring during 15-24 hours until it finally reached a pH of 3.9-4.3.

Then further components are added:
13.6 kg glucidex
0.4 kg pectin
17 g trace element premix
15 g vitamin premix
18.4 g sodium ascorbate
196 g potassium lactate
17 g KCl
100 g NaCl
35 g choline chloride
150 g dibasic potassium phosphate
100 g magnesium subcarbonate
150 g potassium citrate
150 g calcium chloride.
The product is further homogenized and subjected to in line pasteurization and then filled into suitable containers aseptically.

### Example V

### Supplement in powder form

A supplement, in powder form, according to the invention can be obtained by processing a fermented product according to Example I by spray-drying to produce a powder. This powder can be reconstituted with water or a suitable solution to produce the drink, suitable for use.

## Claims

1. Liquid, acid nutrient composition with long shelf life, comprising 0.1-5 wt.% "egg white protein"(EWP), based on the total composition, wherein less than 40 %, preferably less than 10 % and most preferentially less than 2 % of the EWP is denatured, and which nutrient composition contains less than 15 wt.% egg yolk components and less than 20 % by weight whey proteins, based on total protein.

2. Nutrient composition according to Claim 1, which contains 0.01 - 0.7 % by weight conalbumin, based on the total composition.

3. Nutrient composition according to Claim 1 or 2, in the form of a fermented and pasteurised dairy product, in particular a yoghurt drink.

4. Nutrient composition according to Claim 1 or 2, in the form of a complete food, such as a food for administration by tube or catheter, especially into the stomach.

5. Nutrient composition according to one of the preceding claims, based on goats' milk.

6. Nutrient composition according to Claim 1 or 2, in the form of an EWP-containing fruit drink.

7. Nutrient composition according to claim 1 or 2, in the form of an EWP containing fruit drink, containing less than 10 % by weight whey proteins, preferably less than 5 % by weight, based on total protein.

8. Nutrient composition according to any of claims 1-7, containing less than 5 % by weight egg yolk components, preferably less than 0.5 % by weight, based on total protein.

9. Use of EWP or a product derived from EWP in particular in dried form or in the form of a spray dried composition in the preparation of a liquid acid nutrient composition according to any one of claims 1-8.

10. Use of EWP or a product derived from EWP, in particular in dried form or in the form of a spray dried composition, in the preparation of a liquid acid nutrient composition with long shelf life, wherein less than 40 %, preferably less than 10 % and most preferentially less than 2 % of the EWP is denatured, and which nutrient composition contains less than 15 wt.% egg yolk components.

11. Use according to claim 9 or 10, in the preparation of a nutrient composition for the prevention or treatment of infections and/or other inflammatory disorders, free radical-mediated diseases and/or disorders of the digestive tract.

12. Use according to any of claims 9 to 11, wherein the conalbumin concentration in the final composition is 0.01-0.7 % by weight.

13. Use according to one of Claims 9-12, in the preparation of a fermented and pasteurised dairy product, in particular a yoghurt drink.

14. Use according to one of Claims 9-12, in the preparation of a complete food, such as a food for administration by tube or catheter.

15. Use according to one of Claims 9-12, in the preparation of an EWP-containing fruit or vegetable drink.

16. Dry composition, in particular in powder form, suitable for producing a nutrient composition according to one of Claims 1-8 after reconstitution.

17. Method for the preparation of a nutrient composition according to one of Claims 1-8, comprising the reconstitution of a dry composition according to Claim 16.

## Patentansprüche

1. Flüssige saure Nährstoffzusammensetzung mit langer Haltbarkeitszeit, enthaltend 0,1 bis 5 Gew.-% "Eiweißprotein" (EWP), bezogen auf die Gesamtzusammensetzung, wobei weniger als 40 %, vorzugsweise weniger als 10 % und am bevorzugtesten weniger als 2 % des EWP denaturiert sind und die Nährstoffzusammensetzung weniger als 15 Gew.-% Eigelb-Komponenten und weniger als 20 Gew.-% Molkeproteine, bezogen auf das Gesamtprotein, enthält.

2. Nährstoffzusammensetzung nach Anspruch 1, die 0,01 bis 0,7 Gew.-% Conalbumin, bezogen auf die Gesamtzusammensetzung, enthält.

3. Nährstoffzusammensetzung nach Anspruch 1 oder 2 in Form eines fermentierten und pasteurisierten Molkereiproduktes, insbesondere eines Joghurtgetränks.

4. Nährstoffzusammensetzung nach Anspruch 1 oder 2 in Form eines Vollnahrungsmittels, z.B. in Form eines Nahrungsmittels zur Verabreichung mit Hilfe eines Schlauchs oder eines Katheters, speziell in den Magen.

5. Nährstoffzusammensetzung nach einem der vorangehenden Ansprüche auf der Basis von Ziegenmilch.

6. Nährstoffzusammensetzung nach Anspruch 1 oder 2 in Form eines EWP-enthaltenden Fruchtgetränks.

7. Nährstoffzusammensetzung nach Anspruch 1 oder 2 in Form eines EWP-enthaltenden Fruchtgetränks, das weniger als 10 Gew.-%, vorzugsweise weniger als 5 Gew.-% Molkeproteine, bezogen auf das Gesamtprotein, enthält.

8. Nährstoffzusammensetzung nach einem der Ansprüche 1 bis 7, die weniger als 5 Gew.-%, vorzugsweise weniger als 0,5 Gew.-% Eigelb-Komponenten, bezogen auf das Gesamtprotein, enthält.

9. Verwendung von EWP oder eines von EWP stammenden Produktes, insbesondere in getrockneter Form oder in Form einer sprühgetrockneten Zusammensetzung, zur Herstellung einer flüssigen sauren Nährstoffzusammesetzung nach einem der Ansprüche 1 bis 8.

10. Verwendung von EWP oder eines von EWP stammenden Produktes, insbesondere in getrockneter Form oder in Form einer sprühgetrockneten Zusammensetzung, zur Herstellung einer flüssigen sauren Nährstoffzusammensetzung mit langer Haltbarkeitszeit, wobei weniger als 40 %, vorzugsweise weniger als 10 % und am bevorzugtesten weniger als 2 Gew.-% des EWP denaturiert sind und die Nährstoffzusammensetzung weniger als 15 Gew.-% Eigelb-Komponenten enthält.

11. Verwendung nach Anspruch 9 oder 10 zur Herstellung einer Nährstoffzusammensetzung zur Verhütung oder Behandlung von Infektionen und/oder anderen Entzündungskrankheiten, durch Radikale verursachten Krankheiten und/oder Krankheiten des Verdauungstrakts.

12. Verwendung nach einem der Ansprüche 9 bis 11, wobei die Conalbumin-Konzentration in der Endzusammensetzung 0,01 bis 0,7 Gew.-% beträgt.

13. Verwendung nach einem der Ansprüche 9 bis 12 zur Herstellung eines fermentierten und pasteurisierten Molkereiprodukts, insbesondere eines Joghurtgetränks.

14. Verwendung nach einem der Ansprüche 9 bis 12 zur Herstellung eines Vollnahrungsmittels, z.B. eines Nahrungsmittels zur Verabreichung mit Hilfe eines Schlauchs oder eines Katheters.

15. Verwendung nach einem der Ansprüche 9 bis 12 zur Herstellung eines EWP-enthaltenden Frucht- oder Gemüsegetränks.

16. Trockene Zusammensetzung, insbesondere in Pulverform, die nach der Rekonstitution zur Herstellung einer Nährstoffzusammensetzung nach einem der Ansprüche 1 bis 8 geeignet ist.

17. Verfahren zur Herstellung einer Nährstoffzusammensetzung nach einem der Ansprüche 1 bis 8, das die Rekonstitution einer trockenen Zusammensetzung nach Anspruch 16 umfaßt.

## Revendications

1. Composition nutritive acide longue conservation, comprenant 0,1-5 % en poids de "protéine de blanc d'oeuf" (EWP) par rapport à la composition totale, avec moins de 40%, de préférence moins de 10% et de préférence encore moins de 2% de l'EWP dénaturée, ladite composition nutritive contenant moins de 15% en poids de composants de jaune d'oeuf et moins de 20% en poids de protéines de lactoserum par rapport au total de protéine.

2. Composition nutritive selon la revendication 1, **caractérisée en ce qu'**elle contient 0,01 - 0,7 % en poids de conalbumine, par rapport à la composition totale.

3. Composition nutritive selon la revendication 1 ou 2, **caractérisée en ce qu'**elle se présente sous la forme d'un produit laitier fermenté et pasteurisé, en particulier un yaourt à boire.

4. Composition nutritive selon la revendication 1 ou 2, **caractérisée en ce qu'**elle se présente sous la forme d'un aliment complet, tel qu'un aliment à administrer à l'aide d'un tube ou cathéter, particulièrement dans l'estomac.

5. Composition nutritive selon l'une des revendications précédentes, **caractérisée en ce qu'**elle est à base de lait de chèvre.

6. Composition nutritive selon la revendication 1 ou 2, **caractérisée en ce qu'**elle se présente sous la forme d'une boisson aux fruits contenant de l'EWP.

7. Composition nutritive selon la revendication 1 ou 2, **caractérisée en ce qu'**elle se présente sous la forme d'une boisson aux fruits contenant de l'EWP, contenant moins de 10% en poids de protéines de lactoserum, de préférence moins de 5% en poids, par rapport au total de protéine.

8. Composition nutritive selon l'une des revendications 1 à 7, contenant moins de 5% en poids de composants de jaune d'oeuf, de préférence moins de 0,5% en poids, par rapport au total de protéine.

9. Utilisation d'EPW ou d'un produit dérivé d'EPW, en particulier sous une forme sèche ou sous la forme d'une composition sèche pulvérisée, dans la préparation d'une composition nutritive liquide acide selon l'une quelconque des revendications 1 à 8.

10. Utilisation d'EPW ou d'un produit dérivé d'EPW, en particulier sous une forme sèche ou sous la forme d'une composition sèche pulvérisée, dans la préparation d'une composition nutritive liquide acide ayant une longue durée de conservation, dans laquelle moins de 40%, de préférence moins de 10% et de préférence encore moins de 2% d'EWP est dénaturée, ladite composition nutritive contenant moins de 15% en poids de composants de jaune d'oeuf.

11. Utilisation selon la revendication 9 ou 10, dans la préparation d'une composition nutritive pour la prévention ou le traitement d'infections et/ou d'autres disfonctionnements inflammatoires, de maladies véhiculées par des radicaux libres et/ou de disfonctionnements des voies digestives.

12. Utilisation selon l'une des revendications 9 à 11, **caractérisée en ce que** la concentration en conalbumine dans la composition finale est de 0,01-0,7% en poids.

13. Utilisation selon l'une des revendications 9-12, dans la préparation d'un produit laitier fermenté et pasteurisé, en particulier un yaourt à boire.

14. Utilisation selon l'une des revendications 9-12, dans la préparation d'un aliment complet, tel qu'un aliment destiné à être administrée à l'aide d'un tube ou d'un cathéter.

15. Utilisation selon l'une des revendications 9-12, dans la préparation d'une boisson aux fruits ou aux légumes contenant de l'EWP.

16. Composition sèche, en particulier sous forme de poudre, convenant pour la fabrication d'une composition nutritive selon l'une des revendications 1-8 après reconstitution.

17. Procédé pour la préparation d'une composition nutritive selon l'une des revendications 1-8, comprenant la reconstitution d'une composition sèche selon la revendication 16.
